# EUROPÄISCHE PATENTANMELDUNG

(11) **EP 4 722 715 A1**
(43) Veröffentlichungstag der Anmeldung: **08.04.2026**
(21) Anmeldenummer: 25205847.4
(22) Anmeldetag: 30.09.2025
(51) Int. Cl.: G01N 33/00

(54) **ANORDNUNG ZUM FEHLERSICHEREN DETEKTIEREN EINES GASES IN EINEM RAUMBEREICH**

(30) Priorität: 04.10.2024 DE 102024128713
(71) Anmelder: Pilz GmbH & Co. KG, 73760 Ostfildern (DE)
(72) Erfinder: Weishaar, Christoph, 73760 Ostfildern (DE); Monnee, Lambertus, 73760 Ostfildern (DE)
(74) Vertreter: Witte, Weller & Partner Patentanwälte mbB

(57) **Zusammenfassung**

Eine Anordnung zum fehlersicheren Detektieren eines Gases (12) in einem Raumbereich (14) besitzt einen Abstandshaltekörper (16), an dem eine Vielzahl von Gassensoren (18a-18d) mit vordefinierten Abständen relativ zueinander angeordnet sind. Die Gassensoren (18a-18d) sind jeweils sensitiv für das zu detektierende Gas (12). Eine Auswerteeinheit (20) erzeugt auf Basis eines Plausibilitätstests ein Schaltsignal (22), das eine Anwesenheit des Gases (12) in dem Raumbereich (14) repräsentiert. Der Plausibilitätstest setzt ein erstes und zumindest ein zweites Gasdetektionssignal (26a, 26b) in Abhängigkeit von den jeweiligen Haltepositionen (24a, 24b, 24c) der Gassensoren zueinander in Beziehung.

## Beschreibung

Die vorliegende Erfindung betrifft eine Anordnung zum fehlersicheren Detektieren eines Gases in einem Raumbereich, insbesondere zum fehlersicheren Detektieren von Wasserstoff.

Es gibt zahlreiche Situationen, in denen man wissen möchte oder feststellen muss, ob ein bestimmtes Gas in einer bestimmten Menge oder Konzentration in einem Raumbereich vorhanden ist. Beispiele beinhalten Prozesskontrolle und/oder Abgasüberwachung in industriellen Anlagen, Biogasanlagen oder bei gasbetriebenen Fahrzeugen, die Überwachung von Luftschadstoffen, Leckageüberwachung bei Gastanks und Gasleitungssystemen und nicht zuletzt Überwachungen im Rahmen von Maßnahmen zum Explosionsschutz. Letztere können separat oder in Kombination mit anderen hier beispielhaft genannten Überwachungen erfolgen.

Zur Gasdetektion und ggf. Messung einer Gaskonzentration werden geeignete Gassensoren benötigt. Abhängig von der Art des Gases und den Anforderungen an die Selektivität, Sensibilität und Stabilität können verschiedene Messprinzipien zum Einsatz kommen, die chemische und/oder physikalische Eigenschaften eines zu detektierenden Gases ausnutzen. In der Regel erzeugen die Gassensoren ein elektrisches Signal, das von der Gaskonzentration des zu detektierenden Gas abhängt und in einer nachfolgenden Auswerte- und/oder Steuereinheit weiter verarbeitet werden kann.

Beispielsweise offenbart EP 1 879 023 A1 einen Gassensor und dessen Verwendung zur Detektion von Wasserstoff. Der Gassensor weist einen Feldeffekttransistor (FET) mit elektrisch schwebendem Gate und ein Gate mit einer Sensorschicht als sensitiver Schicht zum Detektieren von Wasserstoff (H₂) auf. Die Sensorschicht ist gegenüber dem Messraum durch eine SnO₂ aufweisende Trennschicht getrennt.

DE 11 2016 004 676 T5 offenbart einen Wasserstoffsensor, der in der Lage sein soll, Wasserstoff auch bei normaler oder niedriger Temperatur mit hohem Ansprechvermögen zu detektieren, ohne dass er eine Heizung benötigt. Der Wasserstoffsensor beinhaltet ein Substrat, das eine Isoliereigenschaft aufweist, einen Detektionsfilm, der ein Keramikmaterial und Metallpartikel umfasst, die in dem Keramikmaterial dispergiert sind, und ein Paar Elektroden, die auf einer Fläche des Detektionsfilms angeordnet sind. Die Metallpartikel sind aus einer Legierung von Palladium (Pd) und mindestens einem weiteren Element, das aus einer Elementgruppe von Übergangsmetallen ausgewählt ist, die sich von Palladium unterscheiden.

DE 10 2016 212 117 A1 offenbart eine Vorrichtung und ein Verfahren zum Detektieren einer Leckage in einem Wasserstofftank eines Wasserstoff-Brennstoffzellenfahrzeugs. Auf Basis der Änderung eines Erfassungswertes eines Hochdrucksensors soll eine Leckage aufgrund des Ausfalls eines Magnetventils detektiert werden.

DE 10 2021 201 449 A1 offenbart eine weitere Sensoreinrichtung für ein Wasserstoffspeichersystem eines Kraftfahrzeugs. Das Wasserstoffspeichersystem besitzt einen Wasserstoffspeicher und ein mit dem Wasserstoffspeicher verbundenes Druckminderventil, mit dem der Wasserstoff von einem Tankdruck auf einen niedrigeren Versorgungsdruck für ein Brennstoffzellensystem entspannt werden kann. Die Sensoreinrichtung weist ein Druckentlastungsventil und einen Wasserstoffsensor auf, der dazu ausgebildet ist, Wasserstoff zu detektieren.

Die frühzeitige Detektion einer Leckage an einem gasführenden, gasproduzierenden und/oder gasverarbeitenden System, wie einem Elektrolyseur oder einer Brennstoffzelle, einem Gastank und/oder Gasleitungssystem ist in vielen Fällen sicherheitskritisch, insbesondere im Rahmen von Maßnahmen zum Explosionsschutz. In der Regel wird eine Sicherheitsmaßnahme ausgeführt, sobald ein unerwünschter Gasaustritt bzw. eine unzulässig hohe Gaskonzentration in einer Anlage erkannt wird. Sicherheitsmaßnahmen können die Ausgabe von akustischen und/oder visuellen Alarmsignalen zum Evakuieren des betroffenen Raumbereichs, das Belüften des betroffenen Raumbereichs durch automatisiertes Öffnen von Lüftungsklappen und/oder Türen, ein notfallmäßiges Schließen von Absperrventilen und anderes beinhalten. Häufig führen solche Sicherheitsmaßnahmen dazu, dass ein laufender Prozess, bei dem das Gas verwendet wird, unterbrochen wird. Dies ist nachteilig im Hinblick auf die Produktivität und Effizienz der Anlage, bei der das Gas zum Einsatz kommt. Es ist daher wünschenswert, Fehlalarme im Sinne von objektiv nicht erforderlichen Unterbrechungen eines Normalbetriebs der Anlage zu vermeiden. Ein Fehlalarm in diesem Sinne kann beispielsweise verursacht sein durch eine schleichende Degradierung des Sensors, etwa einem Nachlassen der Sensitivität, oder sogar einem Ausfall, bei dem der Sensor falsche Daten liefert. Andererseits dürfen Produktivität und Effizienz einer Anlage nicht auf Kosten der Sicherheit gehen. Im Zweifel ist daher bei einer Gasdetektion und auch bei einer unklaren Situation eine Sicherheitsmaßnahme notwendig. Dies schließt in vielen Fällen ein, dass eine Anlage abgeschaltet oder anderweitig in einen sicheren Zustand gebracht wird, wenn in der Anlagensteuerung, den mit der Anlagensteuerung verbundenen Sensoren und Aktoren, Kommunikationsverbindungen und/oder an anderer Stelle Fehler auftreten, die zu einer Gefährdung von Menschen und/oder Umwelt führen können.

Vor diesem Hintergrund ist es eine Aufgabe der vorliegenden Erfindung, eine Anordnung der eingangs genannten Art anzugeben, die einerseits eine fehlersichere Detektion eines Gases in einem Raumbereich ermöglicht und die andererseits dazu beiträgt, Fehlalarme zu reduzieren oder gar zu vermeiden.

Gemäß einem Aspekt der vorliegenden Erfindung wird zur Lösung dieser Aufgabe eine Anordnung der eingangs genannten Art vorgeschlagen, mit einem Abstandshaltekörper, mit einer Vielzahl von Gassensoren, die mit definierten Abständen relativ zueinander an dem Abstandshaltekörper gehalten und jeweils sensitiv für das zu detektierende Gas sind, und mit einer Auswerteeinheit, die dazu ausgebildet ist, in Abhängigkeit von den Gassensoren ein Schaltsignal zu erzeugen, das eine Anwesenheit des Gases in dem Raumbereich repräsentiert, wobei der Abstandshaltekörper eine erste Halteposition und zumindest eine zweite Halteposition definiert, die voneinander beabstandet sind, wobei ein erster Gassensor aus der Vielzahl von Gassensoren an der ersten Halteposition sitzt und dazu ausgebildet ist, ein erstes Gasdetektionssignal zu erzeugen, das eine erste Gaskonzentration im Bereich der ersten Halteposition repräsentiert, wobei ein zweiter Gassensor aus der Vielzahl von Gassensoren an der zweiten Halteposition sitzt und dazu ausgebildet ist, ein zweites Gasdetektionssignal zu erzeugen, das eine zweite Gaskonzentration im Bereich der zweiten Halteposition repräsentiert, und wobei die Auswerteeinheit dazu ausgebildet ist, das definierte Schaltsignal auf Basis eines Plausibilitätstests zu bestimmen, der das erste und das zweite Gasdetektionssignal in Abhängigkeit von der ersten Halteposition und der zweiten Halteposition zueinander in Beziehung setzt.

Besonders vorteilhaft wird eine solche Anordnung als Leckagesensor zur Detektion eines unkontrollierten bzw. unbeabsichtigten Gasaustritts aus einem Gasbehälter oder Gasleitungssystem verwendet, insbesondere aus einem Gasbehälter oder Gasleitungssystem, in dem Wasserstoff gelagert ist und/oder transportiert wird. Der Gasbehälter und/oder das Gasleitungssystem können hierbei integrierter Bestandteil eines gasproduzierenden und/oder gasverarbeitenden Systems sein.

Die Anordnung besitzt eine Vielzahl von Gassensoren, die an einem gemeinsamen Abstandshaltekörper angeordnet sind. Der Abstandshaltekörper hält die Gassensoren folglich mit definierten Abständen relativ zueinander an den Haltepositionen. In den bevorzugten Ausführungsbeispielen ist der Abstandshaltekörper ein formstabiles Bauelement, das es möglich macht, die Gassensoren mit definierten Abständen relativ zueinander zu fixieren, bevor die Anordnung als Ganzes zur Gasdetektion in einem Raumbereich installiert wird. Der Abstandshaltekörper verbindet den ersten und den zweiten Gassensor in diesen Ausführungsbeispielen daher zu einem Sensorkopf mit zumindest zwei separaten Gassensoren, die in einem definierten Abstand voneinander angeordnet sind. Bevorzugt ist der Abstandshaltekörper aus Metall, Kunststoff, Faserverbundwerkstoff oder einer Kombination dieser Materialien hergestellt, um eine ausreichende Robustheit gegenüber Umgebungsbedingungen, wie beispielsweise Temperaturschwankungen und/oder mechanischer Belastung, zu gewährleisten. In den bevorzugten Ausführungsbeispielen besitzen die erste und die zweite Halteposition einen definierten Abstand voneinander, der im Bereich von einigen Zentimetern liegt, insbesondere zwischen 1cm und 50cm und vorzugsweise zwischen 5cm und 25cm, wobei die angegebenen Bereichsgrenzen jeweils eingeschlossen sind.

Der definierte Abstand zwischen dem ersten und dem zweiten Gassensor macht es möglich, das erste Gasdetektionssignal und das zweite Gasdetektionssignal determiniert auf Plausibilität zueinander zu überprüfen. Vorteilhaft ermöglicht der definierte Abstand eine räumlich aufgelöste Bestimmung der Gaskonzentration und die Auswerteeinheit ist dazu ausgebildet, das Schaltsignal in Abhängigkeit von der räumlich aufgelösten Gaskonzentration zu erzeugen. In einigen Ausführungsbeispielen kann die Auswerteeinheit dazu ausgebildet sein, das Schaltsignal in Abhängigkeit von einem vordefinierten Verhältnis der ersten und zweiten Gaskonzentration zu erzeugen. Das vordefinierte Verhältnis kann vorteilhaft als Differenz und/oder Quotient der Gasdetektionssignale bzw. der dadurch repräsentierten Gaskonzentrationen bestimmt werden.

Dementsprechend ist die Auswerteeinheit dazu ausgebildet, das definierte Schaltsignal auf Basis eines Plausibilitätstests zu bestimmen, der das erste und das zweite Gasdetektionssignal unter Berücksichtigung der vordefinierten (und damit vorab bekannten) Haltepositionen zueinander in Beziehung setzt. Insbesondere berücksichtigt die Auswerteeinheit eine Erwartungshaltung, die die definierten Haltepositionen des ersten und zweiten Gassensors in der Anordnung repräsentiert. In einigen Ausführungsbeispielen kann die Erwartungshaltung beinhalten, dass das erste Gasdetektionssignal zeitlich vor dem zweiten Gasdetektionssignal auftritt, beispielsweise als Folge davon, dass die zweite Halteposition von einer möglichen Leckagestelle an einem Gastank oder einem Gasleitungssystem weiter entfernt ist als die ersten Halteposition. Vorteilhaft kann mit Hilfe der von einander beabstandet gehaltenen Gassensoren eine Strömungsrichtung des zu detektierenden Gases bestimmt werden und die Auswerteeinheit kann dazu ausgebildet sein, das Schaltsignal in Abhängigkeit von der Strömungsrichtung zu erzeugen. Ein zeitlicher Versatz zwischen dem ersten und dem zweiten Gasdetektionssignal kann alternativ oder ergänzend auch Folge davon sein, dass die Anordnung eine Gasführung aufweist, die dazu ausgebildet ist, ein Leckagegas dem ersten und dem zweiten Gassensor gezielt zu verschiedenen Zeitpunkten zuzuführen. Der Plausibilitätstest kann vorteilhaft den zeitlichen Versatz auswerten.

Alternativ oder ergänzend kann die Erwartungshaltung beinhalten, dass die erste und die zweite Gaskonzentration in einem definierten Verhältnis zueinander stehen. Beispielsweise kann die Erwartungshaltung beinhalten, dass die erste und die zweite Gaskonzentration trotz der voneinander beabstandeten Haltepositionen nach Ablauf einer definierten Zeitspanne weitgehend gleich sind, nämlich innerhalb eines definierten Toleranzintervalls. In anderen Ausführungsbeispielen kann die Erwartungshaltung beinhalten, dass die erste und die zweite Gaskonzentration weitgehend gleiche zeitliche Verläufe zeigen, insbesondere typgleiche Änderungen der Gaskonzentration in einem zeitlichen Verlauf.

Der Plausibilitätstest erfolgt in Kenntnis und unter Berücksichtigung der definierten Haltepositionen und daher in Abhängigkeit von der ersten und der zweiten Halteposition. Er macht es in einigen Ausführungsbeispielen möglich, das Risiko von Fehlalarmen dadurch zu reduzieren, dass die Auswerteeinheit das Schaltsignal nur erzeugt, wenn das erste und das zweite Gasdetektionssignal im Rahmen der Erwartungshaltung konsistent zueinander sind, d.h. bei gemeinsamer Betrachtung konsistent eine Gasdetektion repräsentieren. In weiteren Ausführungsbeispielen kann die Auswerteeinheit das Schaltsignal, das die Anwesenheit des Gases in dem Raumbereich repräsentiert, erzeugen, wenn einer der Gassensoren der Anordnung ein entsprechendes Gasdetektionssignal liefert. In diesen Ausführungsbeispielen ermöglicht die Anordnung eine höhere Fehlersicherheit im Sinne der funktionalen Sicherheit von technischen Anlagen gemäß den Normen EN IEC 61508, EN IEC 61062 und/oder EN ISO 13849. In einigen bevorzugten Ausführungsbeispielen kann die Auswerteeinheit von einem Anwender über eine Konfigurationsschnittstelle konfiguriert werden, um wahlweise eine höhere Verfügbarkeit einer überwachten Anlage im Sinne der Vermeidung von Fehlalarmen oder eine höhere Fehlersicherheit zu erhalten. Die Konfiguration kann sich in einigen Ausführungsbeispielen auch auf die Eigenschaften der Anordnung im Hinblick auf Maßnahmen zum Explosionsschutz erstrecken und somit eine jeweils angepasste Verwendung in Raumbereichen ermöglichen, die einer der Explosionsschutzzonen 1, 2 oder 3 zugeordnet werden.

Insgesamt ermöglicht die neue Anordnung daher einerseits eine fehlersichere Detektion eines Gases in einem Raumbereich und andererseits eine Reduktion von Fehlalarmen. Die Vermeidung von Fehlalarmen führt zu einer erhöhten Akzeptanz bei Anwendern und reduziert Anreize, eine Anlage zur Unterdrückung von Fehlalarmen bzw. zur Erhöhung der Verfügbarkeit zu manipulieren, etwa indem die Überwachung der Gaskonzentration deaktiviert wird. Die oben genannte Aufgabe ist daher vollständig gelöst.

In einer bevorzugten Ausgestaltung der Erfindung besitzen die Gassensoren jeweils eine definierte Gaseintrittsfläche und die Gaseintrittsflächen des ersten und des zweiten Gassensors sind in voneinander verschiedene Raumrichtungen ausgerichtet.

In dieser Ausgestaltung berücksichtigt der Plausibilitätstest vorteilhaft auch die unterschiedlich ausgerichteten Gaseintrittsflächen des ersten und des zweiten Gassensors bzw. sich daraus ergebende Beziehungen zwischen dem ersten und dem zweiten Gasdetektionssignal. Die Ausgestaltung kann beispielsweise vorteilhaft sein, um eine gerichtete Gasströmung und eine weitgehend homogene Gasverteilung in einem Raumbereich voneinander zu unterscheiden und daraus eine Entscheidung abzuleiten, ob das Schaltsignal durch die Auswerteeinheit erzeugt werden soll oder nicht. Des Weiteren kann diese Ausgestaltung vorteilhaft dazu beitragen, einen zeitlichen Versatz zwischen dem ersten und dem zweiten Gasdetektionssignal zu begünstigen. Ein solcher zeitlicher Versatz kann vorteilhaft in dem Plausibilitätstest berücksichtigt sein.

In einer weiteren Ausgestaltung definiert der Abstandshaltekörper eine dritte Halteposition, die von der ersten und der zweiten Halteposition beabstandet ist, wobei die Vielzahl von Gassensoren einen dritten Gassensor aufweist, der an der dritten Halteposition gehalten ist, wobei der dritte Gassensor dazu ausgebildet ist, ein drittes Gasdetektionssignal zu erzeugen, das eine dritte Gaskonzentration im Bereich der dritten Halteposition repräsentiert. Vorzugsweise besitzt der dritte Gassensor eine dritte Gaseintrittsfläche, die in einer dritten Raumrichtung ausgerichtet ist, wobei sich die dritte Raumrichtung von der ersten und zweiten Raumrichtung unterscheidet, in der die Gaseintrittsflächen des ersten und des zweiten Gassensors ausgerichtet sind.

Die dritte Halteposition besitzt einen definierten Abstand zu der ersten und der zweiten Halteposition. Dementsprechend erhöht diese Ausgestaltung die Anzahl der Beziehungen, die die Auswerteeinheit im Rahmen des Plausibilitätstests berücksichtigen kann. Vorzugsweise ist die Auswerteeinheit in einer weiteren Ausgestaltung daher ferner dazu ausgebildet, das definierte Schaltsignal in Abhängigkeit von einem Plausibilitätstest zu bestimmen, der das erste und das dritte Gasdetektionssignal und/oder das zweite und das dritte Gasdetektionssignal zueinander in Beziehung setzt. Die Auswerteeinheit kann das Schaltsignal aufgrund dieser Ausgestaltung auf Basis einer höheren Informationsdichte und daher noch situationsangepasster erzeugen, was dazu beiträgt, Fehlalarme weiter zu reduzieren und zudem eine hohe Fehlersicherheit zu ermöglichen.

Darüber hinaus besitzt diese Ausgestaltung den Vorteil, dass die neue Anordnung noch individueller und variantenreicher konfiguriert werden kann. Daher bietet der dritte Gassensor auch ohne erweiterten Plausibilitätstest einen Vorteil.

In einer weiteren Ausgestaltung weist die Auswerteeinheit eine Konfigurationsschnittstelle auf, die dazu ausgebildet ist, eine Abstimmungsstruktur aus einer Vielzahl von vordefinierten Abstimmungsstrukturen zu konfigurieren, wobei die Vielzahl von vordefinierten Abstimmungsstrukturen eine 1ooX Abstimmungsstruktur und eine 2ooX Abstimmungsstruktur unter Verwendung des ersten und des zweiten Gassensors beinhalten, und wobei die Auswerteeinheit das definierte Schaltsignal in Abhängigkeit von einer konfigurierten Abstimmungsstruktur bestimmt.

Eine Abstimmungsstruktur im Sinne dieser Ausgestaltung definiert eine logische Verknüpfung der Vielzahl von Gassensoren und deren Auswirkung auf die Erzeugung des Schaltsignals in der Auswerteeinheit. Eine 1ooX (1 out of X) Struktur hat zur Folge, dass die Auswerteeinheit das Schaltsignal erzeugt, sobald eines von X Gasdetektionssignalen eine definierte Gaskonzentration in dem Raumbereich anzeigt. Der Buchstabe X steht hier also für die Anzahl der Gassensoren, die im Rahmen der Konfiguration verwendet werden können. Bei zwei Gassensoren steht 1ooX folglich für 1oo2 (1 out of 2). Bei einer 2oo2 (2 out of 2) Abstimmungsstruktur unter Verwendung des ersten und zweiten Gassensors erzeugt die Auswerteeinheit das Schaltsignal nur, wenn das erste und das zweite Gasdetektionssignal die jeweils definierte erste bzw. zweite Gaskonzentration anzeigen. In Ausgestaltungen, in denen die Anordnung einen dritten Gassensor aufweist, beinhaltet die Vielzahl von vordefinierten Abstimmungsstrukturen vorzugsweise ferner zumindest einer der folgenden Abstimmungsstrukturen: 1oo3, 2oo3 und/oder 3oo3. In weiteren vorteilhaften Ausgestaltungen, in denen die Anordnung einen vierten Gassensor aufweist, beinhaltet die Vielzahl von vordefinierten Abstimmungsstrukturen ferner zumindest eine der folgenden Abstimmungsstrukturen: 1oo4, 2oo4, 3oo4 und/oder 4oo4. Die Ausgestaltung erhöht die Einsatzbandbreite der neuen Anordnung und macht es einem Anwender leicht möglich, die Anordnung flexibel an den Einsatzzweck anzupassen. Sie ermöglicht darüber hinaus eine effiziente Lagerhaltung für verschiedene Einsatzbedingungen.

In einer weiteren Ausgestaltung beinhaltet die Anordnung ein schirmartiges Gehäuse, das den Abstandshaltekörper und zumindest einen der Gassensoren überdeckt.

Ein schirmartiges Gehäuse im Sinne dieser Ausgestaltung besitzt eine oder mehrere Flächen, die einen überdeckten Innenraum definieren. Die Fläche kann beispielsweise eine gewölbte, beispielsweise kugelsegmentförmig gewölbte Fläche sein. Prinzipiell kann die Schirmfläche aber In anderen Ausführungsbeispielen eben sein. Das Gehäuse kann mehrere Seitenwände aufweisen, die aneinandergrenzen und einen im Querschnitt polygonen Innenraum definieren. Das Gehäuse besitzt zumindest eine Eintrittsöffnung, insbesondere eine Öffnungsseite, die der Schirmfläche gegenüberliegt. Das Gehäuse kann alternativ oder ergänzend mehrere Eintrittsöffnungen aufweisen, insbesondere seitliche Eintrittsöffnungen in der Schirmfläche. In einigen Ausführungsbeispiele ist die Schirmfläche am Einsatzort der Anordnung, d.h. in der bestimmungsgemäßen Ausrichtung der Anordnung, oberhalb von den Gassensoren positioniert und nach oben hin geschlossen. Die Ausgestaltung besitzt den Vorteil, dass das Gehäuse ein zu detektierendes Gas im Bereich des zumindest einen Gassensors sammelt und auf diese Weise definierte Detektionsverhältnisse begünstigt. Dies wiederum macht es möglich, den Plausibilitätstest mit engeren Toleranzen zu gestalten. In einigen bevorzugten Ausführungsbeispielen überdeckt das schirmartige Gehäuse den ersten und den zweiten Gassensor. Vorzugsweise ist einer der genannten Gassensoren tiefer in dem Gehäuseschirm angeordnet als der andere, d.h. näher zu der Schirmfläche hin positioniert. Die Ausgestaltung trägt zu einer besonders sensitiven und aussagekräftigen Erzeugung des Schaltsignals bei.

In einer weiteren Ausgestaltung weist das schirmartige Gehäuse eine Zwischenwand auf, die das schirmartige Gehäuse in eine erste Kammer und eine zweite Kammer unterteilt, wobei der erste Gassensor in der ersten Kammer angeordnet ist, und wobei der zweite Gassensor in der zweiten Kammer angeordnet ist.

In dieser Ausgestaltung sind der erste und der zweite Gassensor nicht nur von einander beabstandet, sondern zusätzlich in zumindest teilweise voneinander getrennten Kammern angeordnet. Die Kammern schaffen voneinander unterscheidbare Detektionsumgebungen und tragen jeweils zu einer Konzentrationserhöhung bei, was den Plausibilitätstest noch aussagekräftiger macht. Die Ausgestaltung trägt vorteilhaft dazu bei, ein besonders sensitive und zuverlässige Detektionsanordnung zu realisieren.

In einer weiteren Ausgestaltung weist das schirmartige Gehäuse eine Öffnungsseite auf und der erste Gassensor ist zu der Öffnungsseite ausgerichtet.

In dieser Ausgestaltung erreicht ein zu detektierendes Gas den ersten Gassensor determiniert zeitlich früher als den zweiten Gassensor, was den Plausibilitätstest erleichtert und reproduzierbar zuverlässiger macht.

In einer weiteren Ausgestaltung weist der Abstandshaltekörper eine sich verjüngende Außenkontur auf, die die Haltepositionen definiert.

In dieser Ausgestaltung besitzt der Abstandshaltekörper ein erstes Ende, das einen kleineren Außendurchmesser aufweist (mithin "spitzer" ausgebildet ist) als ein gegenüberliegendes zweites Ende. In einigen vorteilhaften Ausführungsbeispielen kann das verjüngte erste Ende näher an der Öffnungsseite angeordnet sein als das zweite Ende. Diese Ausgestaltung besitzt den Vorteil, dass der Gaseintritt in das Gehäuse durch die Form des Abstandshaltekörpers in determinierter Weise begünstigt wird. Die Ausgestaltung trägt zu einer schnellen Gasdetektion vorteilhaft bei. In anderen Ausführungsbeispielen kann das breitere zweite Ende näher an der Öffnungsseite angeordnet sein als das verjüngte erste Ende. Diese Variante begünstigt einen zeitlichen Versatz zwischen dem ersten und dem zweiten Gasdetektionssignal und trägt damit zu einem sehr zuverlässigen und aussagekräftigen Schaltsignal vorteilhaft bei.

In einer weiteren Ausgestaltung beinhaltet der Abstandshaltekörper einen Tetraeder oder eine Pyramide, an dem bzw. an der der erste und der zweite Gassensor angeordnet sind.

Ein Tetraeder ist ein vielflächiger dreidimensionaler Körper mit der geringsten Anzahl an ebenen oder weitgehend ebenen Außenflächen sowie der geringsten Anzahl an Ecken. Er besitzt zudem eine sich verjüngende Außenkontur und profitiert ebenso wie ein pyramidaler Abstandshaltekörper von den zuvor genannten Vorteilen. Der Tetraeder ermöglicht eine besonders vorteilhafte Platzierung von bis zu vier Gassensoren auf den Flächen oder an den Ecken. Mit Hilfe von derartig angeordneten Gassensoren kann ein 3D-Raumvolumen mit einer geringen Anzahl an Gassensoren "rundum" auf Plausibilitätsbeziehungen überwacht werden. Ein pyramidaler Abstandshaltekörper besitzt vergleichbare Vorteile, benötigt allerdings mehr Gassensoren, um das 3D-Raumvolumen gleichermaßen "rundum" auf Plausibilitätsbeziehungen zu überwachen. Beide Ausgestaltungen besitzen den Vorteil, dass die Gassensoren relativ einfach auf vorzugsweise ebenen Flächenabschnitten und/oder an den Ecken platziert werden können. Vorzugsweise sind die Gassensoren in die Flächenabschnitte und/oder Ecken eingelassen, was einen mechanischen Schutz der Gassensoren und damit die Robustheit der Anordnung begünstigt.

In einer weiteren Ausgestaltung beinhaltet der Abstandshaltekörper eine gekrümmte Oberfläche, an der der erste und der zweite Gassensor angeordnet sind.

In dieser Ausgestaltung kann der Abstandshaltekörper beispielsweise eine kugelsegmentförmige, zylindrische oder kreiskegelartige Mantelfläche aufweisen, die die Haltepositionen für die Gassensoren definiert. Diese Ausgestaltung ermöglicht eine sehr effiziente und homogene Verteilung einer Vielzahl von Gassensoren, deren Gaseintrittsflächen in verschiedene Richtungen zeigen und eine Vielzahl von Plausibilisierungen ermöglichen.

In einer weiteren Ausgestaltung beinhalten die Vielzahl von Gassensoren zumindest zwei voneinander typverschiedene Gassensoren, welche aus einer Gruppe ausgewählt sind, die einen elektrochemischen Gassensor, einen Halbleiter-Gassensor, einen katalytischen Gassensor und einen optischen Gassensor, insbesondere einen Infrarot-Gassensor beinhaltet. In einigen Ausführungsbeispielen dieser Ausgestaltung kann der Unterschied im Typ auf Materialeigenschaften der Gassensoren bei ansonsten gleichem physikalischen Messprinzip beruhen.

Elektrochemische Sensoren verwenden eine elektrochemische Zelle, in der eine chemische Reaktion zwischen dem Gas und einer Elektrode ein messbares elektrisches Signal erzeugt. Sie eignen sich gut für präzise Messungen bei niedrigen Konzentrationen. Halbleiter-Gassensoren messen die Änderungen in den elektrischen Eigenschaften eines Halbleitermaterials, die durch Anwesenheit des zu detektierenden Gases verursacht werden. Sie sind relativ kostengünstig und reagieren schnell, können aber durch andere Gase beeinflusst werden. Katalytische Gassensoren besitzen zwei Elektroden, insbesondere in Form von Platinwendeln, die in eine Keramikschicht eingebettet und über eine Brückenschaltung elektrisch verbunden sind. Die Oberfläche der einen Elektrode ist mit einem Katalysator, der Oxidation fördert, aktiviert, die Oberfläche der anderen Elektrode ist inaktiviert. Ein Stromfluss durch die Elektroden erhitzt diese und an der Oberfläche der aktiven Elektrode reagiert der Luftsauerstoff mit dem zu detektierenden Gas. Dadurch steigen Temperatur und Widerstand in der aktiven Elektrode und die Brücke gerät ins Ungleichgewicht, was man messen kann. Optische Gassensoren nutzen das Prinzip der Lichtabsorption oder Lichtstreuung, um eine Gaskonzentrationen zu detektieren. Derartige Gassensoren besitzen eine hohe Genauigkeit und sind oft robust, können aber teuer sein.

In dieser Ausgestaltung besitzt die Anordnung zumindest zwei Gassensoren, deren Detektionsfähigkeit auf unterschiedlichen physikalischen und/oder chemischen Prinzipien und/oder auf unterschiedlichen Materialeigenschaften der sensitiven Flächen beruhen. Die typverschiedenen Gassensoren ermöglichen einen besonders aussagekräftigen Plausibilitätstest und erleichtern die Implementierung einer besonders hohen Fehlersicherheit im Sinne der oben genannten Normen zur funktionalen Sicherheit aufgrund der diversitären Detektionsprinzipien.

In einer weiteren Ausgestaltung weist die Anordnung einen Drehantrieb auf, der eine Drehachse definiert, und die Auswerteeinheit ist dazu ausgebildet, zumindest einen Gassensor mit Hilfe des Drehantriebs um die Drehachse zu drehen.

Vorzugsweise ist die Auswerteeinheit dazu ausgebildet, den Abstandshaltekörper um die Drehachse zu drehen. Die Anordnung dieser Ausgestaltung macht es möglich, dass die Auswerteeinheit die Raumlage des zumindest einen Gassensors verändern kann, um die Richtung eines Gasstroms zu analysieren. Die Anordnung dieser Ausgestaltung kann gewissermaßen nach einer erhöhten Gaskonzentration "schnüffeln". Vorzugsweise ist die Auswerteeinheit dazu ausgebildet, das Schaltsignal in Abhängigkeit von einer aktuellen Drehposition des zumindest einen Gassensors zu erzeugen. Die Ausgestaltung ermöglicht eine noch zielgerichtete Erzeugung des Schaltsignals.

In einer weiteren Ausgestaltung beinhaltet der Plausibilitätstest eine Differenzbildung zwischen dem ersten und dem zweiten Gasdetektionssignal.

Eine Differenzbildung ermöglicht einen sehr schnellen Vergleich zwischen dem ersten und dem zweiten Gasdetektionssignal und begünstigt daher eine kostengünstige Realisierung einer sehr schnell ansprechenden Detektoranordnung.

In einer weiteren Ausgestaltung wertet der Plausibilitätstest einen zeitlichen Abstand zwischen dem ersten und dem zweiten Gasdetektionssignal aus.

Diese Ausgestaltung nutzt die voneinander beabstandeten Haltepositionen für die Gassensoren auf eine sehr vorteilhafte Weise, um eine aussagekräftiges Schaltsignal mit einer geringen Wahrscheinlichkeit für Fehlalarme zu implementieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel der neuen Vorrichtung in einer schematischen Darstellung,
- Fig. 2: eine schematische Darstellung von zeitlichen Verläufen eines ersten und eines zweiten Gasdetektionssignals,
- Fig. 3: eine vereinfachte Darstellung eines weiteren Ausführungsbeispiel von Gassensoren an einem gemeinsamen Abstandshaltekörper, und
- Fig. 4: eine vereinfachte Darstellung eines weiteren Ausführungsbeispiel von Gassensoren an einem gemeinsamen Abstandshaltekörper.

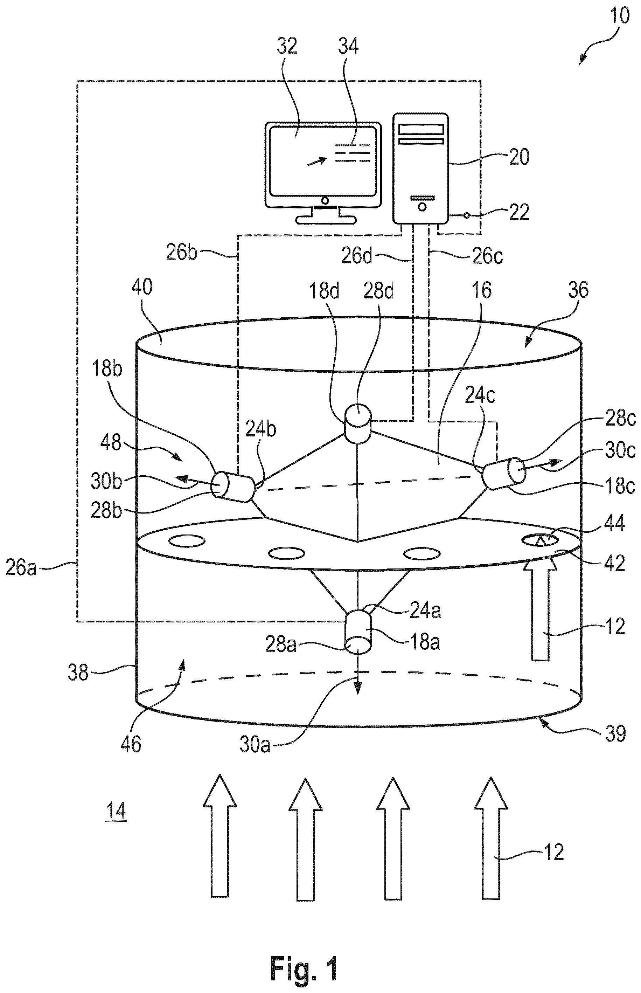

In Fig. 1 ist ein Ausführungsbeispiel der neuen Anordnung in seiner Gesamtheit mit der Bezugsziffer 10 bezeichnet. Die Anordnung 10 ist dazu ausgebildet, ein Gas 12 in einem Raumbereich 14 zu detektieren, und sie kann vorteilhaft verwendet werden, um beispielsweise ein Gasleck an einem Gastank und/oder Gasleitungssystem (hier nicht dargestellt) zu detektieren, um im Fall einer Gasdetektion eine Sicherheitsfunktion auszulösen.

Die Anordnung 10 beinhaltet einen Abstandshaltekörper 16, an dem eine Vielzahl von Gassensoren 18a-18d angeordnet sind. Die Gassensoren 18a-18d sind jeweils sensitiv für das zu detektierende Gas 12. In bevorzugten Ausführungsbeispielen sind die Gassensoren beispielsweise sensitiv für Wasserstoff (H₂). Alternativ oder ergänzend können die Gassensoren 18a-18d auch für andere Gase sensitiv sein. In einigen bevorzugten Ausführungsbeispielen beinhalten die Gassensoren 18a-18d zumindest zwei voneinander typverschiedene Gassensoren, die aus einer Gruppe ausgewählt sind, die einen elektrochemischen Gassensor, einen Halbleiter-Gassensor, einen katalytischen Gassensor und einen Infrarot-Gassensor beinhaltet. In anderen Ausführungsbeispielen sind die Gassensoren 18a-18d typgleiche Gassensoren, insbesondere Halbleiter-Gassensoren, die für Wasserstoff sensitiv sind.

Die Anordnung 10 besitzt ferner eine Auswerteeinheit 20, die dazu ausgebildet ist, ein Schaltsignal 22 in Abhängigkeit von den Gassensoren 18a-18d zu erzeugen. Das Schaltsignal 22 signalisiert einer nachgelagerten Anlagensteuerung (hier nicht dargestellt) eine Gasdetektion in dem Raumbereich 14 und kann vorteilhaft verwendet sein, um eine Sicherheitsmaßnahme in einer Anlage in dem Raumbereich 14 auszulösen, beispielsweise einen optischen und/oder akustischen Alarm, automatisiertes Öffnen von Belüftungsklappen und/oder automatisiertes Schließen von Absperrventilen.

Der Abstandshaltekörper 16 ist in diesem Ausführungsbeispiel ein Tetraeder mit vier Seitenflächen und vier Ecken. In diesem Ausführungsbeispiel definieren die vier Ecken eine erste Halteposition 24a, an der ein erster Gassensor 18a gehalten ist, eine zweite Halteposition 24b, an der ein zweiter Gassensor 18b gehalten ist, eine dritte Halteposition 24c, an der ein dritter Gassensor 18c gehalten ist, und eine vierte Halteposition (hier nicht bezeichnet), an der ein vierter Gassensor 18d gehalten ist. Der erste Gassensor 18a ist dazu ausgebildet, ein erstes Gasdetektionssignal 26a zu erzeugen, das eine erste Gaskonzentration im Bereich der ersten Halteposition 24a repräsentiert. Der zweite Gassensor 18b ist dazu ausgebildet, ein zweites Gasdetektionssignal 26b zu erzeugen, das eine zweite Gaskonzentration im Bereich der zweiten Halteposition 24b repräsentiert. Der dritte Gassensor 18c ist dazu ausgebildet, ein drittes Gasdetektionssignal 26c zu erzeugen, das eine dritte Gaskonzentration im Bereich der dritten Halteposition 24c repräsentiert. Schließlich ist der vierte Gassensor 18d dazu ausgebildet, ein viertes Gasdetektionssignal 26d zu erzeugen, das eine vierte Gaskonzentration im Bereich der vierten Halteposition repräsentiert. Wie man anhand Fig. 1 erkennen kann, sind die Haltepositionen und die dort jeweils angeordneten Gassensoren 18a-18d voneinander beabstandet. Die jeweiligen Abstände zwischen den Gassensoren 18a-18d haben zur Folge, dass die jeweils gemessenen Gaskonzentrationen an den Haltepositionen 24a, 24b, 24c voneinander verschieden sein können, je nachdem ob und wie schnell sich ein zu detektierendes Gas in dem Bereich um den Abstandshaltekörper 16 herum verteilt. Die durch den Abstandshaltekörper 16 definierten und fixierten Abstände zwischen jeweils zwei Gassensoren werden in der Auswerteeinheit vorteilhaft berücksichtigt, wenn die Auswerteeinheit 20 das definierte Schaltsignal 22 auf Basis eines Plausibilitätstests bestimmt, der das erste Gasdetektionssignal 26a und die weiteren Gasdetektionssignale 26b-26d zueinander in Beziehung setzt.

Die Gassensoren 18a-18d besitzen jeweils eine Gaseintrittsfläche 28a, 28b, 28c, 28d. Bevorzugt weisen die Gaseintrittsflächen 28a, 28b, 28c, 28d der Gassensoren 18a-18d in voneinander verschiedene Raumrichtungen. Beispielhaft ist eine erste Raumrichtung, in die die Gaseintrittsfläche 28a des ersten Gassensors 18a zeigt, mit der Bezugsziffer 30a bezeichnet. Eine zweite Raumrichtung, in die die Gaseintrittsfläche 28b des zweites Gassensors 18b zeigt, ist mit der Bezugsziffer 30b bezeichnet. Eine dritte Raumrichtung, in die die Gaseintrittsfläche 28c des dritten Gassensors 18c zeigt, ist mit der Bezugsziffer 30c bezeichnet. Die unterschiedlichen Raumrichtungen können in einigen Ausführungsbeispielen in dem Plausibilitätstest der Auswerteeinheit 20 vorteilhaft berücksichtigt sein.

Mit der Bezugsziffer 32 ist hier ein Monitor bezeichnet, der eine Konfigurationsschnittstelle 32 für die Auswerteeinheit 20 repräsentiert. Die Konfigurationsschnittstelle 32 macht es in bevorzugten Ausführungsbeispielen möglich, eine Abstimmungsstruktur aus einer Vielzahl von vordefinierten Abstimmungsstrukturen 34 auszuwählen. Eine ausgewählte Abstimmungsstruktur definiert, wie die Auswerteeinheit 20 das Schaltsignal in Abhängigkeit von den Gasdetektionssignalen 26a-26d der Gassensoren 18a-18d bestimmt. Beispielsweise kann die Auswerteeinheit 20 das Schaltsignal 22 erzeugen, wenn das Gasdetektionssignal eines der Gassensoren 18a-18d eine Gaskonzentration über einem definierten Schwellenwert anzeigt, unabhängig davon, ob die weiteren Gasdetektionssignale ebenfalls eine solche Gaskonzentration signalisieren. In diesem Fall erzeugt die Auswerteeinheit 20 das Schaltsignal in einer 1oo4 (1 out of 4) Abstimmungsstruktur. Eine 1oo4 Abstimmungsstruktur bietet eine hohe Sicherheit im Hinblick auf eine Gasdetektion und macht es möglich, einen hohen Safety Integrity Level (SIL) im Sinne der Normen EN IEC 61508 und EN IEC 61062 und/oder einen hohen Performance Level (PL) im Sinne der Norm EN ISO 13849 für eine überwachte Anlage zu implementieren. Des Weiteren eignet sich eine solche Abstimmungsstruktur sehr gut für Maßnahmen im Rahmen des Explosionsschutzes in einer Zone 0, d.h. einer Zone mit einer hohen Explosionsgefahr. Nachteilig ist, dass eine solche Abstimmungsstruktur eine höhere Wahrscheinlichkeit für Fehlalarme zur Folge hat. Ein Fehlalarm in diesem Sinn ist die Erzeugung des Schaltsignals 22, obwohl dies in der konkreten Situation objektiv nicht notwendig gewesen wäre, etwa weil eine Gaskonzentration über dem definierten Schwellenwert lokal sehr begrenzt vorhanden war und diese Gaskonzentration sich bei der weiteren Verteilung des Gases im Raum unter den definierten Schwellenwert abschwächt und somit nur sporadisch und in einem sehr kurzen Zeitintervall auftrat.

Die Anordnung 10 bietet daher in bevorzugten Ausführungsbeispielen die Möglichkeit, eine von mehreren vordefinierten Abstimmungsstrukturen 34 über die Konfigurationsschnittstelle 32 auszuwählen. In Ausführungsbeispielen mit vier Gassensoren kann die Konfigurationsschnittstelle 32 vorteilhaft folgende Abstimmungsstrukturen zur Auswahl stellen: 1oo4, 2oo4, 3oo4, 4oo4. Bei einer ausgewählten 2oo4 Abstimmungsstruktur erzeugt die Auswerteeinheit 20 das Schaltsignal 22 erst, wenn zumindest zwei der vier Gassensoren 18a-18d eine Gaskonzentration über dem definierten Schwellenwert signalisieren. Die 2oo4 Abstimmungsstruktur reduziert daher die Wahrscheinlichkeit für Fehlalarme und trägt zu einer höheren Verfügbarkeit der überwachten Anlage bei. Eine 4oo4 Abstimmungsstruktur bietet eine noch geringere Wahrscheinlichkeit für Fehlalarme und eine entsprechend höhere Verfügbarkeit der überwachten Anlage, allerdings zu Lasten der Fehlersicherheit im Sinne der Normen EN IEC 61508, EN IEC 61062 und/oder EN ISO 13849.

In einigen Ausführungsbeispielen kann die Konfigurationsschnittstelle 32 für den Anwender, insbesondere einen Monteur der Anordnung 10 zugänglich gemacht sein. In anderen Ausführungsbeispielen kann die Konfigurationsschnittstelle 32 dem Hersteller der Anordnung 10 vorbehalten und für Anwender unzugänglich und/oder verborgen sein.

In dem dargestellten Ausführungsbeispiel besitzt die Anordnung 10 ein schirmartiges Gehäuse 36 mit einer in diesem Fall geschlossenen Mantelfläche 38, einer Öffnungsseite 39 und einem geschlossenen Boden 40. In anderen Ausführungsbeispielen kann das Gehäuse eine in bestimmungsgemäßer Montage am Einsatzort nach oben gewölbte Schirmfläche aufweisen, etwa nach Art eines nach oben gehaltenen Regenschirms.

Der Abstandshaltekörper 16 mit den Gassensoren 18a-18d ist in dem Innenraum des Gehäuses 36 angeordnet, der von der Mantelfläche 38 und dem Boden 40 gebildet wird. In dem Gehäuse 36 ist in diesem Ausführungsbeispiel ferner eine (bevorzugte, aber optionale) Zwischenwand 42 mit Durchgangsöffnungen 44 angeordnet. Die Zwischenwand 42 bildet in dem schirmartigen Gehäuse 36 eine erste Kammer 46 und eine zweite Kammer 48. Wie man in Fig. 1 sehen kann, ist der Gassensor 18a in diesem Ausführungsbeispiel in der ersten Kammer 46 angeordnet, während die Gassensoren 18b-18d in der zweiten Kammer 48 sitzen. Ein zu detektierendes Gas gelangt hier konstruktionsbedingt über die Öffnungsseite 39 des Gehäuses 36 in den Innenraum und erreicht zunächst den Gassensor 18a, der näher zu der Öffnungsseite 39 liegt. Erst mit einer zeitlichen Verzögerung kann sich das zu detektierende Gas 12 in die zweite Kammer 48 ausbreiten und von den dort angeordneten Gassensoren 18b-18d detektiert werden.

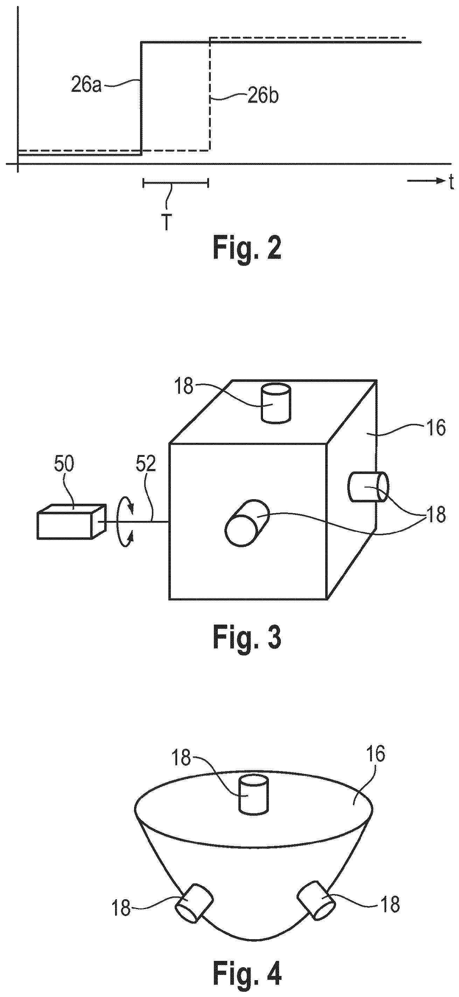

Dementsprechend erzeugt der Gassensor 18a das Gasdetektionssignal 26a zeitlich früher als die weiteren Gassensoren 18b-18d die jeweiligen weiteren Gasdetektionssignale erzeugen können. Die zeitliche Verzögerung T zwischen dem Gasdetektionssignal 26a und einem weiteren Gasdetektionssignal 26b ist in Fig. 2 schematisch dargestellt und wird in bevorzugten Ausführungsbeispielen von der Auswerteeinheit 20 im Rahmen des Plausibilitätstests ausgewertet, um das Schaltsignal 22 in Abhängigkeit von den Gasdetektionssignalen zu bestimmen.

In einigen Ausführungsbeispielen kann die Auswerteeinheit 20 alternativ oder ergänzend die unterschiedlichen Raumrichtungen 30a, 30b, 30c der Gassensoren 18a-18d im Rahmen des Plausibilitätstests berücksichtigen, da auch die unterschiedlichen Raumrichtungen 30a, 30b, 30c zu individuellen zeitlichen Verzögerungen zwischen den Gasdetektionssignalen 26a-26d führen können. Dies ist insbesondere der Fall, wenn zumindest ein Gassensor (hier der Gassensor 18a) mit seiner Gaseintrittsfläche 28a zu der Öffnungsseite 39 zeigt, wohingegen zumindest ein weiterer Gassensor (hier die Gassensoren 18b-18d) mit ihrer Gaseintrittsfläche von der Öffnungsseite 39 abgewandt sind.

In Ausführungsbeispielen kann die Auswerteeinheit 20 ferner die zeitlichen Beziehungen zwischen den weiteren Gasdetektionssignalen 26b-26d untereinander berücksichtigen. Beispielsweise ist für die in Fig. 1 dargestellte Anordnung als Erwartungshaltung anzunehmen, dass ein Gas 12 zuerst von dem Gassensor 18a detektiert wird und erst mit zeitlicher Verzögerung T von den weiteren Gasdetektoren 18b-18d. Des Weiteren ist als Erwartungshaltung anzunehmen, dass die weiteren Gasdetektoren 18b-18d das Gas 12 weitgehend zeitgleich detektieren, wenn die Durchgangsöffnungen 44 in der Zwischenwand 42 eine weitgehend homogene Ausbreitung des Gases 12 in die zweite Kammer 48 ermöglichen. Es ist aber in anderen Ausführungsbeispielen denkbar, dass die Zwischenwand 42 und/oder die Verteilung der weiteren Gassensoren 18b-18d gezielt dazu ausgebildet sind, weitere zeitliche Verzögerungen zu begünstigen.

In einigen Ausführungsbeispielen können die Gassensoren 18a-18d voneinander verschiedene Schwellenwerte haben und die Gasdetektionssignale daher bei individuell unterschiedlichen Ansprechschwellen erzeugen. Die Auswerteeinheit 20 kann vorteilhaft dazu ausgebildet sein, daraus zu erwartende Beziehungen zwischen den Gasdetektionssignalen im Rahmen des Plausibilitätstests auszuwerten und das Schaltsignal 22 in Abhängigkeit von einer sich daraus ergebenden Erwartungshaltung zu erzeugen.

Der in Fig. 1 dargestellte Tetraeder ist ein Beispiel für einen Abstandshaltekörper 16, der eine sich verjüngende Außenkontur aufweist. Ein weiteres Ausführungsbeispiel eines solchen Abstandshaltekörpers ist in Fig. 4 dargestellt, in der der Abstandshaltekörper 16 eine gekrümmte Oberfläche beinhaltet, an der Gassensoren 18 angeordnet sind. Gleiche Bezugszeichen bezeichnen hier dieselben Elemente wie zuvor.

Ein Abstandshaltekörper mit einer sich verjüngenden Außenkontur begünstigt einen gerichteten Gasstrom entlang des Abstandshaltekörpers von einem oder mehreren stromaufwärts gehaltenen Gassensoren zu einem oder mehreren stromabwärts gehaltenen Gassensoren. In bevorzugten Ausführungsbeispielen berücksichtigt die Auswerteeinheit eine aus dem Strömungsverlauf abgeleitete Erwartungshaltung in dem Plausibilitätstest. Vorteilhaft kann die sich verjüngende Außenkontur des Abstandshaltekörpers gasdicht geschlossen sein und/oder gasleitende Elemente oder Kanäle aufweisen, um den Strömungsverlauf in definierter Weise zu beeinflussen. Prinzipiell ist es aber in anderen Ausführungsbeispielen denkbar, dass der Abstandshaltekörper eine andere Form aufweist, wie beispielsweise anhand Fig. 3 dargestellt ist.

In einigen Ausführungsbeispielen kann der Plausibilitätstest in der Auswerteeinheit eine Differenzbildung zwischen zwei oder mehreren Gasdetektionssignalen beinhalten. Ein Differenzsignal ermöglicht eine sehr schnelle Signalauswertung, da zeitlich synchrone und zueinander konsistente Gasdetektionssignale bei einer Differenzbildung zu Nullsignalen führen und etwaige Abweichungen zwischen den Gasdetektionssignalen in einem Differenzsignal leicht detektiert werden können.

Wie in Fig. 3 schematisch angedeutet ist, kann die Anordnung in einigen Ausführungsbeispielen zumindest einen Drehantrieb 50 aufweisen, der eine Drehachse 52 definiert. Die Auswerteeinheit kann in diesen Ausführungsbeispielen dazu ausgebildet sein, den Abstandshaltekörper 16 und/oder einzelne Gassensoren 18a-18d um die Drehachse 52 zu drehen, insbesondere um die Richtung eines detektierten Gasstroms zu bestimmen bzw. zu "erschnüffeln".

## Patentansprüche

1. Anordnung zum fehlersicheren Detektieren eines Gases (12) in einem Raumbereich (14), insbesondere zum fehlersicheren Detektieren von Wasserstoff,
- mit einem Abstandshaltekörper (16),
- mit einer Vielzahl von Gassensoren (18a-18d), die mit definierten Abständen relativ zueinander an dem Abstandshaltekörper (16) gehalten und jeweils sensitiv für das zu detektierende Gas (12) sind, und
- mit einer Auswerteeinheit (20), die dazu ausgebildet ist, in Abhängigkeit von den Gassensoren (18a-18d) ein Schaltsignal (22) zu erzeugen, das eine Anwesenheit des Gases (12) in dem Raumbereich (14) repräsentiert,
wobei der Abstandshaltekörper (16) eine erste Halteposition (24a) und zumindest eine zweite Halteposition (24b) definiert, die voneinander beabstandet sind,
wobei ein erster Gassensor (18a) aus der Vielzahl von Gassensoren (18a-18d) an der ersten Halteposition (24a) sitzt und dazu ausgebildet ist, ein erstes Gasdetektionssignal (26a) zu erzeugen, das eine erste Gaskonzentration im Bereich der ersten Halteposition (24a) repräsentiert,
wobei ein zweiter Gassensor (18b) aus der Vielzahl von Gassensoren (18a-18d) an der zweiten Halteposition (24b) sitzt und dazu ausgebildet ist, ein zweites Gasdetektionssignal (26b) zu erzeugen, das eine zweite Gaskonzentration im Bereich der zweiten Halteposition (24b) repräsentiert, und
wobei die Auswerteeinheit (20) dazu ausgebildet ist, das definierte Schaltsignal (22) auf Basis eines Plausibilitätstests zu bestimmen, der das erste und das zweite Gasdetektionssignal (26a, 26b) in Abhängigkeit von der ersten Halteposition (24a) und der zweiten Halteposition (24b) zueinander in Beziehung setzt.

2. Anordnung nach Anspruch 1, wobei die Gassensoren (18a-18d) jeweils eine definierte Gaseintrittsfläche (28a-28c) besitzen, und wobei die Gaseintrittsflächen (28a, 28b) des ersten und des zweiten Gassensors (18a, 18b) in voneinander verschiedene Raumrichtungen (30a, 30b) ausgerichtet sind.

3. Anordnung nach Anspruch 1 oder 2, wobei der Abstandshaltekörper (16) eine dritte Halteposition (24c) definiert, die von der ersten und der zweiten Halteposition (24a, 24b) beabstandet ist, und wobei die Vielzahl von Gassensoren (18a-18d) einen dritten Gassensor (18c) aufweist, der an der dritten Halteposition (24c) gehalten ist, wobei der dritte Gassensor (18c) dazu ausgebildet ist, ein drittes Gasdetektionssignal (26c) zu erzeugen, das eine dritte Gaskonzentration im Bereich der dritten Halteposition (24c) repräsentiert.

4. Anordnung nach Anspruch 3, wobei die Auswerteeinheit (20) ferner dazu ausgebildet ist, das definierte Schaltsignal (22) in Abhängigkeit von einem Plausibilitätstest zu bestimmen, der das erste und das dritte Gasdetektionssignal (26a, 26c) und/oder das zweite und das dritte Gasdetektionssignal (26b, 26c) zueinander in Beziehung setzt.

5. Anordnung nach Anspruch 1 bis 4, wobei die Auswerteeinheit (20) eine Konfigurationsschnittstelle (32) aufweist, die dazu ausgebildet ist, eine Abstimmungsstruktur aus einer Vielzahl von vordefinierten Abstimmungsstrukturen (34) auszuwählen, wobei die Vielzahl von vordefinierten Abstimmungsstrukturen (34) eine 1ooX Abstimmungsstruktur und eine 2ooX Abstimmungsstruktur unter Verwendung des ersten und des zweiten Gassensors (18a, 18b) beinhalten, und wobei die Auswerteeinheit (20) das definierte Schaltsignal 822) in Abhängigkeit von einer ausgewählten Abstimmungsstruktur bestimmt.

6. Anordnung nach einem der Ansprüche 1 bis 5, ferner mit einem schirmartigen Gehäuse (36), das den Abstandshaltekörper (16) und zumindest einen der Gassensoren (18a-18d) überdeckt.

7. Anordnung nach Anspruch 6, wobei das schirmartige Gehäuse (36) eine Zwischenwand (42) aufweist, die das schirmartige Gehäuse (36) in eine erste Kammer (46) und eine zweite Kammer (48) unterteilt, wobei der erste Gassensor (18a) in der ersten Kammer (46) angeordnet ist, und wobei der zweite Gassensor (18b) in der zweiten Kammer (48) angeordnet ist.

8. Anordnung nach Anspruch 6 oder 7, wobei das schirmartige Gehäuse (36) eine Öffnungsseite (39) aufweist, und wobei der erste Gassensor (18a) zu der Öffnungsseite (39) ausgerichtet ist.

9. Anordnung nach einem der Ansprüche 1 bis 8, wobei der Abstandshaltekörper (16) eine sich verjüngende Außenkontur aufweist, die die Haltepositionen (24a-24c) definiert.

10. Anordnung nach einem der Ansprüche 1 bis 9, wobei der Abstandshaltekörper (16) einen Tetraeder beinhaltet, an dem zumindest der erste und der zweite Gassensor (18a, 18b) angeordnet sind.

11. Anordnung nach einem der Ansprüche 1 bis 10, wobei der Abstandshaltekörper (16) eine gekrümmte Oberfläche beinhaltet, an der zumindest der erste und der zweite Gassensor (18a, 18b) angeordnet sind.

12. Anordnung nach einem der Ansprüche 1 bis 11, wobei die Vielzahl von Gassensoren (18a-18d) zumindest zwei voneinander typverschiedene Gassensoren beinhalten, welche aus einer Gruppe ausgewählt sind, die einen elektrochemischen Gassensor, einen Halbleiter-Gassensor, einen katalytischen Gassensor und einen Infrarot-Gassensor beinhaltet.

13. Anordnung nach einem der Ansprüche 1 bis 12, wobei die Anordnung einen Drehantrieb (50) aufweist, der eine Drehachse (52) definiert, und wobei die Auswerteeinheit (20) dazu ausgebildet ist, zumindest einen Gassensor (18a-18d) mit Hilfe des Drehantriebs (50) um die Drehachse (52) zu drehen.

14. Anordnung nach einem der Ansprüche 1 bis 13, wobei der Plausibilitätstest einen zeitlichen Abstand zwischen dem ersten und dem zweiten Gasdetektionssignal (26a, 26b) auswertet.

15. Verwendung einer Anordnung nach einem der Ansprüche 1 bis 14 als Leckagesensor zur Detektion eines unkontrollierten Gasaustritts aus einem Gasbehälter oder Gasleitungssystem.
